# EUROPEAN PATENT SPECIFICATION

(11) **EP 3 021 118 B1**
(45) Date of publication and mention of the grant of the patent: **12.09.2018**
(21) Application number: 14823083.2
(22) Date of filing: 20.05.2014
(51) Int. Cl.: C12Q 1/6858, C12Q 1/6872, C12Q 1/70

(54) **METHOD FOR IDENTIFYING SPECIES USING MOLECULAR WEIGHTS OF NUCLEIC ACID CLEAVAGE FRAGMENTS**
VERFAHREN ZUR IDENTIFIKATION VON SPEZIES ANHAND DES MOLEKULARGEWICHTS VON NUKLEINSÄURESPALTUNGSFRAGMENTEN
PROCÉDÉ D'IDENTIFICATION D'UNE ESPÈCE À L'AIDE DE MASSES MOLÉCULAIRES DE FRAGMENTS DE CLIVAGE D'ACIDES NUCLÉIQUES

(30) Priority: 09.07.2013 US 201361844089 P
(43) Date of publication of application: 18.05.2016
(73) Proprietor: Tech-Knowhow Corporation, Taipei City 11041 (CN)
(72) Inventor: LING, Chingtung, Taipei City 11041 (CN); CHUNG, Muhua, Taipei City 11041 (CN); CHENG, Juitung, Taipei City 11041 (CN)
(74) Representative: Scholz, Volker
(86) International application number: PCT/CN2014/077870
(87) International publication number: WO 2015/003531

(56) References cited:
- CN-A- 1 977 053
- CN-A- 101 680 872
- CN-A- 103 060 431
- US-A1- 2004 121 315
- US-A1- 2005 142 584
- F. VON WINTZINGERODE ET AL: "Base-specific fragmentation of amplified 16S rRNA genes analyzed by mass spectrometry: A tool for rapid bacterial identification", PROCEEDINGS OF THE NATIONAL ACADEMY OF SCIENCES, vol. 99, no. 10, 14 May 2002 (2002-05-14), pages 7039-7044, XP055338459, US ISSN: 0027-8424, DOI: 10.1073/pnas.102165899
- HALL T A ET AL: "Base composition analysis of human mitochondrial DNA using electrospray ionization mass spectrometry: A novel tool for the identification and differentiation of humans", ANALYTICAL BIOCHEMISTRY, ELSEVIER, AMSTERDAM, NL, vol. 344, no. 1, 1 September 2005 (2005-09-01), pages 53-69, XP027204597, ISSN: 0003-2697 [retrieved on 2005-08-14]

## Description

### TECHNICAL FIELD OF THE INVENTION

The present invention relates to a method for species identification, and more particularly to a method for species identification by using molecular weights of nucleic acid cleavage fragments.

### BACKGROUND OF THE INVENTION

Organism species identification or allogenic identification is mostly conducted by the DNA sequencing method. When this method is clinically used, the difficulties of complicated processes, inefficiency, and high cost are encountered. Another method, restriction fragment length polymorphism (RFLP), can be used, but the accuracy of this method is not high enough, since the nucleic acid cleavage fragments having similar lengths and cannot easily be distinguished during electrophoreses, or the nucleic acid cleavage fragments having the same lengths but different sequences can not separated by electrophoreses. Although many other methods have subsequently been developed, such as the DNA microarrays, the real-time PCR and the next-generation DNA sequencing method, the technical instabilities existing in these technologies lead to uncertain outcomes (eg: the DNA microarrays and the real-time PCR) and high costs (eg: the next-generation DNA sequencing method). When the genotyping of the DNA microarray of human papillomavirus (HPV) is taken as an example, due to the non-specific hybridization reaction resulting from the regions having the high similarity of DNA sequences, the incorrect detection of the designed DNA probes is caused. Moreover, due to the limitations of the detection types of the original product designs, the high variability caused by the development of the virus has made the uncertainty of the conventional methods an urgent problem.

### SUMMARY OF THE INVENTION

The purpose of the present invention is to provide a method for species identification by using molecular weights of nucleic acid cleavage fragments, rather than using electrophoreses or probe hybridization reactions. The stability and accuracy of the present method is high and is not affected by non-specific hybridization, which causes incorrect determination. The identification of nucleic acid cleavage fragments is very accurate, and the slight difference of a single base can be detected. Since different molecules have different molecular weights, the nucleic acid cleavage fragments having the same lengths but different sequences can be detected by the method provided by the present invention. In addition, the method of the present invention has simple processes, low cost, and high efficiency.

To achieve the above object, the present invention provides a method for species identification by using molecular weights of nucleic acid cleavage fragments, comprising steps of:
(S10) performing a polymerase chain reaction by using at least a pair of specific primers to amplify a nucleic acid sequence of a to-be-identified species;
(S20) performing a nucleic acid cleavage reaction by using at least a nuclease to cleave the nucleic acid sequence of the to-be-identified species, so as to generate multiple to-be-tested nucleic acid cleavage fragments having different molecular weights;
(S30) measuring the molecular weights of the to-be-tested nucleic acid cleavage fragments by using a mass spectrometer;
(S40) comparing the molecular weight of each of the to-be-tested nucleic acid cleavage fragments of the to-be-identified species with molecular weights of multiple known nucleic acid cleavage fragments of a known species prestored in a database;
(S50) determining one of the to-be-tested nucleic acid cleavage fragments to be identical to one of the known nucleic acid cleavage fragments when a difference of the molecular weights between the one of the to-be-tested nucleic acid cleavage fragments and the one of the known nucleic acid cleavage fragments is lower than a specific Dalton value; and
(S60) calculating a ratio N/M of a number N of the to-be-tested nucleic acid cleavage fragments, which are determined to be identical to the known nucleic acid cleavage fragments, relative to a total number M of the known nucleic acid cleavage fragments of the known species, wherein the ratio N/M represents similarity of the nucleic acid sequences between the to-be-identified species and the known species.

In accordance with a further feature of an embodiment of the present invention, the specific Dalton value is 2 Daltons.

In accordance with a further feature of an embodiment of the present invention, the method further comprising the following steps after step (S60):
(S71) randomly selecting a greater ratio N/M from a plurality of the ratios N/M of the multiple known species in the database as a center of a high similarity cluster, and randomly selecting a lower ratio N/M as a center of a low similarity cluster;
(S72) calculating differences between each of the ratios N/M of all of the known species and the center of the high similarity cluster, and differences between each of the ratios N/M of all of the known species and the center of the low similarity cluster;
(S73) assigning one of the known species to the high similarity cluster if the difference between the ratio N/M of the one of the known species and the center of the high similarity cluster is lower than the difference between the ratio N/M of the one of the known species and the center of the low similarity cluster; on the contrary, assigning one of the known species to the low similarity cluster if the difference between the ratio N/M of the one of the known species and the center of the low similarity cluster is lower than the difference between the ratio N/M of the one of the known species and the center of the high similarity cluster;
(S74) calculating an average of the ratios N/M of all of the known species in the high similarity cluster, followed by using the average as the new center of the high similarity cluster; and calculating an average of the ratios N/M of all of the known species in the low similarity cluster, followed by using the average as the new center of the low similarity cluster;
(S75) recalculating the differences between each of the ratios N/M of all of the known species and the new center of the high similarity cluster, and the differences between each of the ratios N/M of all of the known species and the new center of the low similarity cluster;
(S76) reassigning one of the known species to the high similarity cluster if the difference between the ratio N/M of the one of the known species and the new center of the high similarity cluster is lower than the difference between the ratio N/M of the one of the known species and the new center of the low similarity cluster; on the contrary, reassigning one of the known species the low similarity cluster if the difference between the ratio N/M of the one of the known species and the new center of the low similarity cluster is lower than the difference between the ratio N/M of the one of the known species and the new center of the high similarity cluster; and
(S77) determining the known species in the high similarity cluster to be the to-be-identified species, and determining the known species in the low similarity cluster not to be the to-be-identified species when the known species reassigned to the high similarity cluster and the known species reassigned to the low similarity cluster are identical to the previous known species assigned to the high similarity cluster and the previous known species assigned to the low similarity cluster of the step (S73);
   wherein when the known species reassigned to the high similarity cluster and the known species reassigned to the low similarity cluster are not identical to the previous known species assigned to the high similarity cluster and the previous known species assigned to the low similarity cluster of the step (S73), the steps of (S74), (S75), and (S76) are repeated until the known species reassigned to the high similarity cluster and the known species reassigned to the low similarity cluster are identical to the previous known species assigned to the high similarity cluster and the previous known species assigned to the low similarity cluster of the step (S76); and then the known species in the high similarity cluster is determined to be the to-be-identified species, and the known species in the low similarity cluster is determined not to be the to-be-identified species;
   wherein the number of the known species in the database in step (S40) is more than 2.

In accordance with a further feature of an embodiment of the present invention, the method, further comprising steps of:
comparing the molecular weight of each of the known nucleic acid cleavage fragments of one of the known species prestored in the database with the molecular weight of each of the known nucleic acid cleavage fragments of another similar one of the known species prestored in the database prior to the step (S40), so as to determine any repeated known nucleic acid cleavage fragment between the two known species; and
omitting comparing each of the to-be-tested nucleic acid cleavage fragments of the to-be-identified species with the repeated known nucleic acid cleavage fragment in the step (S40).

In accordance with a further feature of an embodiment of the present invention, the one of the known species and the similar one of the known species are both selected from the high similarity cluster when comparing the molecular weight of each of the known nucleic acid cleavage fragments of the one of the known species prestored in the database with the molecular weight of each of the known nucleic acid cleavage fragments of the similar one of the known species prestored in the database.

In accordance with a further feature of an embodiment of the present invention, the nucleic acid sequence is a DNA sequence.

In accordance with a further feature of an embodiment of the present invention, the method further comprising a step of: performing a transcription reaction to transcribe the DNA sequence into an RNA sequence prior to the step (S20).

In accordance with a further feature of an embodiment of the present invention, the nuclease is an RNase.

In accordance with a further feature of an embodiment of the present invention, the RNase is RNase A, which cleaves the RNA sequence at U sites.

In accordance with a further feature of an embodiment of the present invention, the to-be-identified species is a microorganism.

In accordance with a further feature of an embodiment of the present invention, the microorganism is a bacterium or a virus.

In accordance with a further feature of an embodiment of the present invention, the to-be-identified species is an animal.

In accordance with a further feature of an embodiment of the present invention, the to-be-identified species is *Homo sapiens.*

### BRIEF DESCRIPTION OF THE DRAWINGS

The invention described herein is with reference to the accompanying drawings, used as examples only, wherein:
FIG. 1 is a flowchart of a method for species identification in accordance with an embodiment of the present invention;
FIG. 2 is a flowchart of a cluster analysis in a method for species identification in accordance with an embodiment of the present invention;
FIG. 3 is a flowchart of a method for species identification including steps of omitting repetition in accordance with another embodiment of the present invention; and
FIGs. 4A-4B are case distribution graphs of HPV virus types, respectively illustrating the results of HPV case identification by using a sequencing method and a method for species identification in accordance with an embodiment of the present invention.

### DETAILED DESCRIPTION OF THE INVENTION

Now refer to the following non-limiting embodiments for further understanding the present invention. It should be appreciated that the following embodiments are merely exemplary, and should not be regarded as the limitations of the present invention. In this embodiment, the identification of human papillomavirus (HPV) is used to explain the method provided by the present invention. However, this method can also be applied to the identification of other species, such as microorganisms (bacteria or viruses), animals, and *Homo sapiens* (for example, the detection of gene mutations).

### Human papillomavirus (HPV):

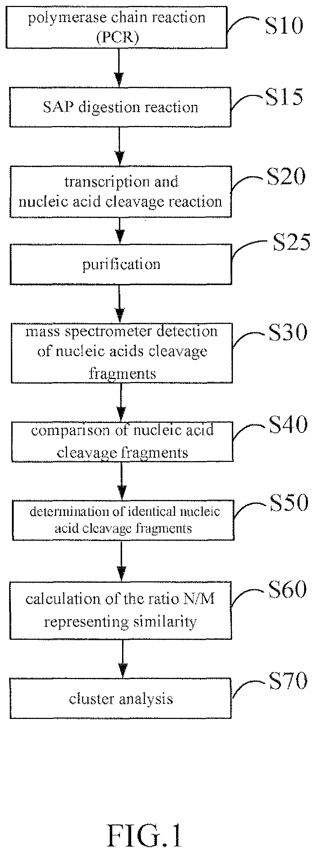

Human papillomavirus (HPV) is a DNA virus, belonging to the papillomavirus family and the *papillomaviridae* genus. This virus infects the human skin and the mucosal tissue. About 170 types of HPV are identified at this time. Some types of HPV cause warts or cancer after invading the human body, but others do not cause any symptoms. Around 30-40 types of HPV are transmitted to the genitals and the surrounding skin through sexual activity, and some of them can cause genital warts. If an individual is repeatedly infected with the high-risk types of HPV which do not cause any wart symptoms, the precancerous lesion or even the invasive cancer may be developed. According to the research studies, 99.7% of cervical cancers are caused by HPV infection. In accordance with the risk degree, for example, HPV-6, HPV-11, HPV-41, HPV-42, HPV-43, and HPV-44 are classified into the low-risk types of HPV, and HPV-16, HPV-18, HPV- 31, and HPV-33 are classified into the high-risk types of HPV, which may easily cause cervical cancer. Although HPV is the main cause of cervical cancer, not all of HPV will cause cervical intraepithelial neoplasia (CIN) and cervical cancer. Thus, the identification of HPV types is crucial in clinical diagnosis. However, the multiple type infection is a common phenomenon in HPV epidemiology, and an individual may be infected with different types of HPV during different time periods, so a specimen may contain multiple types of HPV, increasing the difficulty in the identification of HPV. The technical feature of the present invention provides a method which is capable of identifying multiple types of viruses in a single specimen. Please refer to FIG. 1, which is a flowchart of a method for species identification in accordance with an embodiment of the present invention. The method provide by the present invention includes the following steps: (S10) the polymerase chain reaction (PCR), (S15) the SAP (shrimp alkaline phosphatase) digestion reaction, (S20) the transcription and the nucleic acid cleavage reaction, (S25) the purification, (S30) the mass spectrometer detection of nucleic acids cleavage fragments, (S40) the comparison of nucleic acid cleavage fragments, (S50) the determination of identical nucleic acid cleavage fragments, (S60) the calculation of the ratio N/M representing the similarity, (S70) the cluster analysis, and (S35, S40') the steps of omitting repetition, as shown in FIG. 1.

### Polymerase chain reaction (PCR):

The DNA is extracted by using a commercially available DNA extraction kit, such as QIAGEN Blood Mini Kit®. Firstly, the cells collected from the patient's endothelial mucus are dissolved in the lysis buffer, and the DNA is released from the cell. Under certain conditions, when passing through the column provided by the extraction kit, the DNA binds to a silica-gel membrane inside the column and remains on the membrane. At this time, the membrane is washed with ethanol and the wash buffer, and then is centrifuged to remove impurities. The DNA is finally eluted out with pure water, and the DNA is extracted (please refer to the manual of the DNA extraction kit for the detailed extraction procedures). The aforementioned DNA extraction method is only an embodiment. A variety of DNA extraction methods can be utilized in the method for species identification of the present invention, and, therefore, the extraction method should not be used to limit the scope of the claims of the present invention.

After the DNA extraction, the PCR is used to detect the DNA fragments of HPV, in the present invention, MY09 primer and MY11 primer are used to amplify a specific fragment in the gene of L1 caspid protein. The fragment has a low variability, and, hence, the primers can identify and amplify the fragment in the DNA of different types of HPV. Furthermore, the primers also include the T7 sequence, which is used as the promoter for the subsequent transcription. The forward primer sequence is:

5'-CAGTAATACGACTCACTATAGGGAGAAGGCTGCMCAGGGWCATAAY
AATGG-3

The reverse primer sequence is:

5'-CGATTTAGGTGACACTATAGAAGAGAGGCTCGTCCMARRGGAWACTG

ATC-3 '

Thereafter, a commercially available PCR kit is used to perform the PCR, such as Takara Ex Taq Hot Start Version Kit®. The total volume of each reaction is 25 uL, and the concentration and the volume of each reagent and each specimen are as shown in Table 1, wherein the 10X buffer contains 20 nM Mg²⁺.

**Table 1:**

| | 10X buffer | dNTP | Ex-Taq | MY09/11+T7 primers | specimen DNA | pure water |
|---|---|---|---|---|---|---|
| Concentration | -- | 2.5 nM | 5 units/uL | final concentration 0.2-1.0 uM | different specimens have different concentrations | -- |
| volume | 2.5 uL | 2 uL | 0.125 uL | | 5 uL | Water volume is different based on primer volume |

The concentration and the volume of each reagent and each specimen are prepared in accordance with the above table. The PCR is performed for 35 cycles, and the PCR products are obtained after the reaction. The temperatures of the denature reaction, the annealing reaction and the extension reaction are as shown in Table 2.

**Table 2:**

| | denature | annealing | extension |
|---|---|---|---|
| Temp | 94°C | 60°C | 72°C |

Additionally, the same PCR method is performed to amplify a fragment of in beta-actin gene, and the PCR product of the fragment of in beta-actin gene is used as the positive control group for monitoring and confirming the experiment process and the product quality. After the PCR is complete, the PCR products are obtained. A capillary electrophoresis is used to confirm that the PCR products contain the DNA fragments, such as E-gene HDA GT12 Capillary Electrophoresis®. Then the commercially available analysis software is used to analyze the results, such as QUAxcel Screening Gel®, as shown in Table 3. The aforementioned polymerase chain reaction (PCR) is an exemplary embodiment. A variety of PCR can be utilized in the method for species identification of the present invention, and, therefore, the extraction method should not be used to limit the scope of the claims of the present invention.

**Table 3:**

| fragment number | fragment base pairs | Concentration |
|---|---|---|
| 1 | 13 | n/a |
| 2 | 108 | 4.89 |
| 3 | 499 | 31.11 |
| 4 | 600 | n/a |

In Table 3, the fragment 3 is the DNA fragment in the gene of L1 caspid protein.

### SAP (shrimp alkaline phosphatase) digestion reaction:

Shrimp alkaline phosphatase (SAP) is used to remove the phosphate on DNA 5' end, for preventing DNA 5' end from connecting 3' end of the same DNA fragment, so as to keep the DNA fragment linear. The concentrations and the volumes of the reagents used in the SAP digestion reaction, as shown in Table 4.

**Table 4:**

| reaction agent | final concentration of each reaction | volume (ul) | |
|---|---|---|---|
| | | volume each reaction | volume of 96-well microplate |
| RNase-free Water | | 3.4 | 408.0 |
| SAP (1U/ul) | 0.04U/ul | 0.6 | 72.0 |
| totol volume | | 4 | 480.0 |

The concentration and the volume of each reagent are prepared to form the SAP solution in accordance with the above table. 4ul of the SAP solution is added into the 384-well microplate, and then 2.5ul of the PCR product is added. After sealed with a adhesive film, the 384-well microplate is shaken, then centrifuged under 1000 RPM for one minute, heated to 37°C for 20 minutes, heated to 85°C for 10 minutes, and cooled down to 4°C for storing the product obtained from the SAP digestion reaction. The SAP digestion reaction is an exemplary embodiment. A variety of SAP digestion reactions can be utilized in the method for species identification of the present invention, and, therefore, the extraction method should not be used to limit the scope of the claims of the present invention.

### Transcription and nucleic acid cleavage reaction:

T7 DNA & RNA polymerase is used to initiate *in vitro* transcription at T7 promoter. In the reaction deoxy-cytidine triphosphate (dCTP), uridine triphosphate (UTP), adenosine triphosphate (ATP), and guanosine triphosphate (GTP) are used as the materials for polymerization for synthesizing the mixed product of deoxyribonucleic acid and ribonucleic acid. At the same time, RNase A performs the RNA nucleic acid cleavage reaction on the product at the U (uridine) sites, and the RNA product is cleaved into nucleic acid cleavage fragments having different sizes. Since viruses belonging to the same virus type have identical or very similar nucleic acid sequences, after the nucleic acid sequences from the viruses belonging to the same virus type undergo the nucleic acid cleavage reaction of RNase, the identical or similar sizes of the nucleic acid cleavage fragments are generated from the viruses belonging to the same virus type.

The above embodiment of the transcription and the nucleic acid cleavage reaction is shown as follows. In accordance with the concentration and the volume of each reagent as shown in Table 5, the transcription-cleavage solution is prepared.

| reaction reagent | final concentration of each reaction | volume (ul) | |
|---|---|---|---|
| | | volume each reaction | volume of 96-well microplate |
| RNase-free water | NA | 1.08 | 129.6 |
| 5X polymerase buffer | 1X | 0.9 | 108.0 |
| cleavage mix | NA | 0.12 | 14.4 |
| DTT 100mM | 5.6mM | 0.14 | 16.8 |
| T7 RNA polymerase | 4.4 U/reaction | 0.22 | 26.4 |
| RNase A 0.08mg/ml | 0.7ng/ul | 0.04 | 4.8 |
| Total Volume | | 2.5 | 300.0 |

2.5 ul of the transcription-cleavage solution is added into the 384-well microplate, and then 2ul of the product obtained from the SAP digestion reaction is added into the 384-well microplate. After sealed with a adhesive film, the 384-well microplate is shaken, then centrifuged under 1000 RPM for one minute, heated to 37 °C for 3 minutes, and cooled down to 4°C for storing the obtained product. The transcription and the nucleic acid cleavage reaction are an exemplary embodiment. A variety of transcription and nucleic acid cleavage reactions can be utilized in the method for species identification of the present invention, and, therefore, the extraction method should not be used to limit the scope of the claims of the present invention.

### Purification:

6 mg of clean resin (Clean Resin) is filled in a dimple plate by using a spatula, and is left to stand for 20-30 minutes to dry slightly. 21.5ul of water and 7 ul of the transcription-cleavage product are added into the 384-well microplate, and then centrifuged for 30 seconds. The dimple plate is placed upside down onto the 384-well plates, so that the cleaning resin is filled in each hole. After sealed with an adhesive film, the 384-well microplate is shaken, then centrifuged under 1000 RPM for one minute. After the protestation reaction for 15 minutes, the purified product is centrifuged under 3200 g for 5 minutes, and ready for being dispensed on chips. The purification step is an exemplary embodiment. A variety of purification steps can be utilized in the method for species identification of the present invention, and, therefore, the extraction method should not be used to limit the scope of the claims of the present invention.

### Mass spectrometer detection of nucleic acid cleavage fragments:

The purified product is dispensed on the chips (SpectroCHIP®, America Sequenom Inc.) containing the substrate by using nanodispenser (nanodispenser®, United States Sequenom Inc.), and the nucleic acid cleavage fragments are excited to fly in the vacuum electric field by using the time of flight mass spectrometer. The molecular weight of each nucleic acid cleavage fragment is obtained by the sensor capturing the signal of each nucleic acid cleavage fragment. Afterward, the nucleic acid cleavage fragments prestored in a database are compared with the nucleic acid cleavage fragments of the to-be-identified species, and the identification results are determined.

### Database of nucleic acid cleavage fragments:

In the conventional method, when electrophoreses separating nucleic acid cleavage fragments having different sizes are taken as an example, two nucleic acid cleavage fragments between which the size difference is more than 1-5 bases at best can be separated, and two nucleic acid cleavage fragments having identical base numbers but different sequences can not be separated in electrophoreses. In the present invention, the mass spectrometer is utilized to measure molecular weight of each nucleic acid cleavage fragment, instead of using the electrophoresis to determine the size of each nucleic acid cleavage fragment. Different nucleotides have their respective molecular weights, as shown in Table 6:

**Table 6:**

| nucleotide | molecular weight(Dalton,g/mol) |
|---|---|
| nucleotide triphosphates (average molecular weight = 499.5) | |
| ATP | 507.2 |
| CTP | 483.2 |
| GTP | 523.2 |
| UTP | 484.2 |

| deoxy-nucleotide triphosphate (average molecular weight = 487.0) | |
|---|---|
| dATP | 491.2 |
| dCTP | 467.2 |
| dGTP | 507.2 |
| dTTP | 482.2 |

Therefore, not only do nucleic acid cleavage fragments having different sizes have different molecular weights, but so also do nucleic acid cleavage fragments having different sequences have different molecular weight. It can be seen that the method for detecting nucleic acid cleavage fragments by using the mass spectrometer can precisely distinguish two nucleic acid cleavage fragments between which the size difference is less than 1 base, and can even distinguish two nucleic acid cleavage fragments having the same lengths but different sequences. Thus, the precision thereof is far better the conventional method by using electrophoreses.

The sequences of HPV virus types may be available from the NIAID (National Institute of Allergy and Infectious Disease) web site of the NIH (National Institutes of Health) of the United States (http://pave.niaid.nih.gov/index.html#prototypes?type= human).

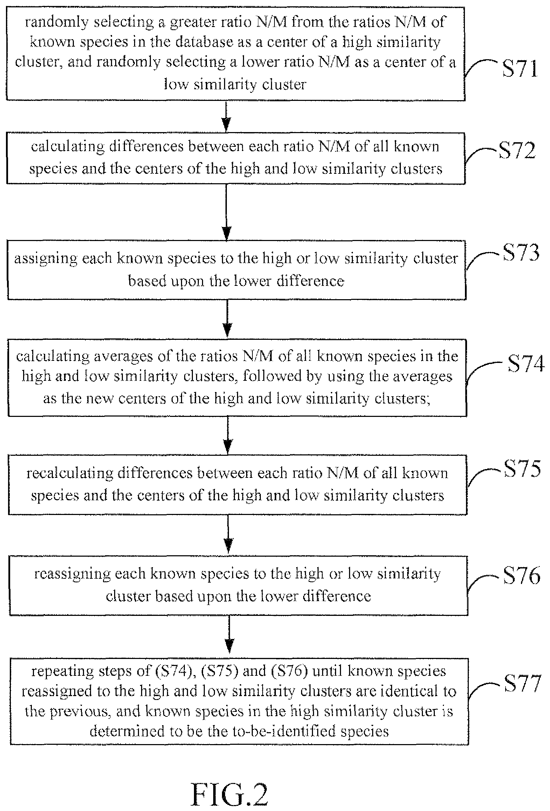

Through the molecular weight of each nucleotide in Table 6 and the characteristic that RNase cleaves RNA at uracil (U) sites (corresponding to thymine (T) sites of DNA of HPV), after HPV undergoes the nucleic acid cleavage fragment reaction, the molecular weights of the nucleic acid cleavage fragments can be calculated. The molecular weight of each nucleic acid cleavage fragments of each HPV virus type is stored to establish the database of the molecular weights of the nucleic acid cleavage fragments of HPV, as shown in Table 7A and 7B. FIG. 2 shows the sizes of the nucleic acid cleavage fragments of each HPV virus type in the database in the present invention.

**Table 7A:**

| virus type | HPV001 | HPV002 | HPV003 | HPV004 | HPV005 | HPV006 | HPV007 | HPV008 | HPV009 | HPV010 |
|---|---|---|---|---|---|---|---|---|---|---|
| fragment 1 | 1891.192 | 1851.167 | 1811.143 | 1891.192 | 1811.143 | 1851.167 | 1923.19 | 1811.143 | 1851.167 | 1867.166 |
| fragment 2 | 1963.215 | 1867.166 | 1867.166 | 1907.191 | 1891.192 | 1931.216 | 1931.216 | 1851.167 | 1907.191 | 1891.192 |
| fragment 3 | 2019.239 | 1891.192 | 1923.19 | 1931,216 | 1907.191 | 1963.215 | 1947.216 | 1923.19 | 1923.19 | 1923.19 |
| fragment 4 | 2212.376 | 1907.191 | 1963.215 | 1947.216 | 1923.19 | 2332.449 | 1963.215 | 1947.216 | 1947.216 | 1963.215 |
| fragment 5 | 2252.4 | 1923.19 | 2003.24 | 1963.215 | 1947.216 | 2380.447 | 2196.376 | 1987.24 | 1963.215 | 2003.24 |
| fragment 6 | 2276.425 | 1963.215 | 2236.401 | 1979.214 | 1963.215 | 2509.587 | 2212.376 | 2196.376 | 1987.24 | 2196.376 |
| fragment 7 | 2332.449 | 2003.24 | 2268.399 | 1987.24 | 1979.214 | 2565.611 | 2220.402 | 2252.4 | 2196.376 | 2220.402 |
| fragment 8 | 2348.449 | 2100.328 | 2292.425 | 2019.239 | 1987.24 | 2613.609 | 2300.451 | 2292.425 | 2236.401 | 2268.399 |
| fragment 9 | 2364.448 | 2156.352 | 2332.449 | 2220.402 | 2252.4 | 2637.634 | 2316.45 | 2348.449 | 2276.425 | 2316.45 |
| fragment 10 | 2565.611 | 2172.351 | 2364.448 | 2236.401 | 2308.424 | 2693.658 | 2348.449 | 2525.586 | 2332.449 | 2324.423 |
| fragment 11 | 2637.634 | 2252.4 | 2501.561 | 2276.425 | 2316.45 | 2758.747 | 2364.448 | 2597.609 | 2348.449 | 2364.448 |
| fragment 12 | 2758.747 | 2292.425 | 2814.771 | 2332.449 | 2332.449 | 3159.98 | 2565.611 | 2605.635 | 2541.585 | 2501.561 |
| fragment 13 | 2854.796 | 2324.423 | 2838.796 | 2581.61 | 2364.448 | 3649.288 | 2597.609 | 2621.635 | 2838.796 | 2565.611 |
| fragment 14 | 3264.055 | 2332.449 | 3143.981 | 2597.609 | 2597.609 | 3762.376 | 2653.633 | 2653.633 | 2926.819 | 2613.609 |
| fragment 15 | 3585.238 | 2348.449 | 3561.213 | 2693.658 | 2661.659 | 3778.375 | 2894.82 | 2661.659 | 3256.029 | 2814.771 |
| fragment 16 | 3794.374 | 2501.561 | 3585.238 | 2718.723 | 2886.794 | 3850.398 | 2902.794 | 2814.771 | 3890.423 | 2950.844 |
| fragment 17 | 3874.424 | 2581.61 | 3834.399 | 3722.351 | 2894.82 | 3913.2 | 3384.076 | 2830.77 | 3913.2 | 3175.979 |
| fragment18 | 3913.2 | 2653.633 | 3874.424 | 3913.2 | 2926.819 | 5921.705 | 3593.264 | 2894.82 | 4227.659 | 3200.005 |
| fragment 19 | 3914.448 | 2693.658 | 3913.2 | 3994.497 | 3159.98 | | 3913.2 | 2902.794 | 4790.03 | 3601.238 |
| fragment 20 | 3930.448 | 2814.771 | 5127.213 | | 3913.2 | | 5537.511 | 3529.215 | 4942.102 | 3913.2 |
| fragment 21 | 4757.978 | 2838.796 | 5624.494 | | 3930.448 | | 7752.865 | 3913.2 | | 4107.585 |
| fragment 22 | 4878.052 | 3913.2 | | | 4846.054 | | | 3914.448 | | 5640.494 |
| fragment 23 | | 4123.584 | | | 6789.261 | | | 7006.423 | | 5761.607 |
| fragment 24 | | 5745.608 | | | | | | | | 7576.767 |
| fragment 25 | | | | | | | | | | |

**Table 7B:**

| virus type | HPV011 | HPV012 | HPV013 | HPV014 | HPV015 | HPV016 | HPV017 | HPV018 | HPV019 | HPV020 |
|---|---|---|---|---|---|---|---|---|---|---|
| fragment 1 | 1907.191 | 1907.191 | 1963.215 | 1891.192 | 1931.216 | 1851.167 | 1931.216 | 1851.167 | 1891.192 | 1907.191 |
| fragment 2 | 1931.216 | 1931.216 | 1995.214 | 1907.191 | 1963.215 | 1931.216 | 1979.214 | 1891.192 | 1907.191 | 1923.19 |
| fragment 3 | 1963.215 | 1947.216 | 2252.4 | 1947.216 | 2252.4 | 1971.241 | 1995.214 | 1923.19 | 1931.216 | 1931.216 |
| fragment 4 | 2003.24 | 1963.215 | 2268.399 | 1963.215 | 2276.425 | 2180.377 | 2276.425 | 1931.216 | 1963.215 | 1947.216 |
| fragment 5 | 2212.376 | 1971.241 | 2276,425 | 1971.241 | 2348.449 | 2236.401 | 2292.425 | 1963.215 | 2228.375 | 1971.241 |
| fragment 6 | 2268.399 | 2196.376 | 2292,425 | 2220.402 | 2549.611 | 2276.425 | 2332.449 | 1987.24 | 2236.401 | 1979.214 |
| fragment 7 | 2332.449 | 2236.401 | 2380.447 | 2236.401 | 2621.635 | 2300.451 | 2525.586 | 2003.24 | 2260.426 | 2196.376 |
| fragment 8 | 2364.448 | 2260.426 | 2541.585 | 2260.426 | 2653.633 | 2332.449 | 2597.609 | 2220.402 | 2276.425 | 2236.401 |
| fragment 9 | 2380.447 | 2276.425 | 2621.635 | 2276.425 | 2677.658 | 2348.449 | 2637.634 | 2236.401 | 2316.45 | 2276.425 |
| fragment 10 | 2637.634 | 2332.449 | 2653.633 | 2324.423 | 2878.821 | 2364.448 | 2677.658 | 2268.399 | 2661.659 | 2597.609 |
| fragment 11 | 2653.633 | 2613,609 | 2661.659 | 2661.659 | 2910.82 | 2509.587 | 2709.657 | 2292.425 | 2677.658 | 2621.635 |
| fragment 12 | 2709.657 | 2637.634 | 2830.77 | 2693.658 | 2926.819 | 2541.585 | 2854.796 | 2308.424 | 2830.77 | 2661.659 |
| fragment 13 | 2798.772 | 2653.633 | 2854.796 | 2878.821 | 3609.264 | 2565.611 | 2910.82 | 2348.449 | 2870.795 | 2693.658 |
| fragment 14 | 2838.796 | 2677.658 | 2870.795 | 2886.794 | 3898.449 | 2597.609 | 3898.449 | 2846.77 | 2910.82 | 2886.794 |
| fragment 15 | 2926.819 | 2854.796 | 3336.078 | 3513.215 | 3913.2 | 2653.633 | 3913.2 | 2910.82 | 2926.819 | 2910.82 |
| fragment 16 | 3159.98 | 2910.82 | 3569.239 | 3818.4 | 4725.98 | 2774.746 | 4171.635 | 2942.818 | 2934.845 | 2950.844 |
| fragment 17 | 3649.288 | 2942.818 | 3913.2 | 3913.2 | 8339.261 | 2870.795 | 4822.028 | 2950.844 | 2966.844 | 3513.215 |
| fragment 18 | 3778.375 | 3913.2 | 4203.633 | 3914.448 | | 2878.821 | 7198.52 | 3665.288 | 3159.98 | 3913.2 |
| fragment 19 | 3890.423 | 3970.472 | 4798.003 | 4163.609 | | 2990.869 | | 3913.2 | 3665.288 | 5745.608 |
| fragment 20 | 3913.2 | 4750.005 | 4942.102 | 4782.004 | | 3159.98 | | 4509.857 | 3913.2 | 5905.706 |
| fragment 21 | 4669.956 | 6693.212 | | 4998.126 | | 3384.076 | | | 3970.472 | |
| fragment 22 | 5945.731 | | | | | 3665.288 | | | 4163.609 | |
| fragment 23 | | | | | | | | | | |
| fragment 24 | | | | | | | | | | |
| fragment 25 | | | | | | | | | | |

### Data analysis:

The molecular weight of each to-be-tested nucleic acid cleavage fragment of the to-be-identified species is compared with the molecular weights of multiple known nucleic acid cleavage fragments of the known species prestored in the database. When the difference of the molecular weights between one nucleic acid cleavage fragment of the to-be-identified HPV and one known nucleic acid cleavage fragment is lower than a specific tolerable error (the specific tolerable error is set to be 2 Daltons in the embodiment), two nucleic acid cleavage fragments are determined to be identical. Afterward, a ratio N/M is determined, which is defined as a number N (as the numerator of the ratio) of the to-be-tested nucleic acid cleavage fragments, which are determined to be identical to the known nucleic acid cleavage fragments, relative to the total number M (as the denominator of the ratio) of the known nucleic acid cleavage fragments of the known species. The ratio N/M represents the similarity of the nucleic acid sequences between the to-be-identified species and the known species.

Please refer to Table 8A and Table 8B, which are examples of the virus identification results, and show the similarity ratios between each known species and the to-be-identified species when the method for species identification of the present invention is applied to the identification of HPV virus types. The similarity ratios of Patient 1 to HPV006, HPV070, and HPV075 are respectively 88.89%, 82.61%, and 60.87%, which respectively represents 88.89%, 82.61%, and 60.87% of the nucleic acid cleavage fragments 1 of HPV006, HPV070 and HPV075 identical to the nucleic acid cleavage fragments of HPV carried by patient. Therefore, HPV006 is the most possible HPV virus type of patient 1 and followed by HPV070. The possibility of HPV075 is lower.

**Table 8A:**

| patient 1 | | | | patient 2 | | | |
|---|---|---|---|---|---|---|---|
| virus type | N/M(%) similarity ratio | cluster | N/M(%) similarity ratio after omitting repetition | virus type | N/M(%) similarity ratio | cluster | N/M(%) similarity ratio after omitting repetition |
| HPV006 | 88.89 | ○ | 83.33 | HPV061 | 85 | ○ | 80 |
| HPV070 | 82.61 | ○ | 76.47 | HPV130 | 42.11 | X | 21.43 |
| HPV075 | 60.87 | X | 47.06 | HPV035 | 41.67 | X | 17.65 |
| HPV130 | 57.89 | X | 38.46 | HPV026 | 36.84 | X | 7.69 |
| HPV004 | 57.89 | X | 42.86 | HPV098 | 36.36 | X | 12.5 |
| HPV133 | 57.14 | X | | HPV001 | 36.36 | X | |
| HPV076 | 56 | X | | HPV072 | 35.29 | X | |
| HPV011 | 54.55 | X | | HPV075 | 34.78 | X | |
| HPV145 | 54.55 | X | | HPV151 | 33.33 | X | |
| HPV067 | 54.17 | X | | HPV011 | 31.82 | X | |
| HPV037 | 52.63 | X | | HPV150 | 31.58 | X | |
| HPV018 | 50 | X | | HPV093 | 30 | X | |
| HPV042 | 50 | X | | HPV031 | 30 | X | |
| HPV016 | 48 | X | | HPV009 | 30 | X | |
| HPV039 | 47.83 | X | | HPV013 | 30 | X | |

**Table 8B:**

| patient 3 | | | | patient 4 | | | |
|---|---|---|---|---|---|---|---|
| virus type | N/M(%) similarity ratio | cluster | N/M(%) similarity ratio after omitting repetition | virus type | N/M(%) similarity ratio | cluster | N/M(%) similarity ratio after omitting repetition |
| HPV006 | 88.89 | ○ | 83.33 | HPV061 | 65 | ○ | 53.33 |
| HPV070 | 73.91 | ○ | 64.71 | HPV130 | 47.37 | X | 28.57 |
| HPV042 | 57.14 | X | 40 | HPV009 | 45 | X | 26.67 |
| HPV133 | 57.14 | X | 40 | HPV014 | 42.86 | X | 29.41 |
| HPV075 | 56.52 | X | 41.18 | HPV004 | 42.11 | X | 26.67 |
| HPV011 | 54.55 | X | | HPV072 | 41.18 | X | |
| HPV145 | 54.55 | X | | HPV038 | 40 | X | |
| HPV067 | 54.17 | X | | HPV075 | 39.13 | X | |
| HPV037 | 52.63 | X | | HPV084 | 38.89 | X | |
| HPV004 | 52.63 | X | | IIPV133 | 38.1 | X | |
| HPV130 | 52.63 | X | | HPV012 | 38.1 | X | |
| HPV076 | 52 | X | | HPV037 | 36.84 | X | |
| HPV035 | 50 | X | | HPV150 | 36.84 | X | |
| HPV016 | 48 | X | | HPV071 | 36.84 | X | |
| HPV049 | 47.83 | X | | HPV001 | 36.36 | X | |

### Cluster analysis:

Since the multiple type infection is a common phenomenon in HPV epidemiology, which means that an individual infected with different types of HPV. The individual may be infected with different types of HPV during different time periods. Thus, a specimen may contain multiple types of HPV, increasing the difficulty in the identification of HPV. The present invention provides a method to resolve this problem. In the method, each HPV virus type in the database is compared with the virus type of the to-be-identified HPV. The virus type(s) in the high similarity cluster is/are separated from the database. The virus type(s) in the high similarity cluster is/are the single or multiple virus types of the infection. When there is only a single HPV virus type in the high similarity cluster, the infection is the single type infection. When there are multiple HPV types in the high similarity cluster, the infection is the multiple type infection.

Please refer to FIG. 2, which is a flowchart of the cluster analysis in a method for species identification in accordance with an embodiment of the present invention. The following is a screening method (S70) of the high similarity cluster, including the steps of:
(S71) randomly selecting a greater ratio N/M from a plurality of the ratios N/M of the multiple known species in the database as a center of a high similarity cluster, and randomly selecting a lower ratio N/M as a center of a low similarity cluster;
(S72) calculating differences between each of the ratios N/M of all of the known species and the center of the high similarity cluster, and differences between each of the ratios N/M of all of the known species and the center of the low similarity cluster;
(S73) assigning one of the known species to the high similarity cluster if the difference between the ratio N/M of the one of the known species and the center of the high similarity cluster is lower than the difference between the ratio N/M of the one of the known species and the center of the low similarity cluster; on the contrary, assigning one of the known species to the low similarity cluster if the difference between the ratio N/M of the one of the known species and the center of the low similarity cluster is lower than the difference between the ratio N/M of the one of the known species and the center of the high similarity cluster;
(S74) calculating an average of the ratios N/M of all of the known species in the high similarity cluster, followed by using the average as the new center of the high similarity cluster; and calculating an average of the ratios N/M of all of the known species in the low similarity cluster, followed by using the average as the new center of the low similarity cluster;
(S75) recalculating the differences between each of the ratios N/M of all of the known species and the new center of the high similarity cluster, and the differences between each of the ratios N/M of all of the known species and the new center of the low similarity cluster;
(S76) reassigning one of the known species to the high similarity cluster if the difference between the ratio N/M of the one of the known species and the new center of the high similarity cluster is lower than the difference between the ratio N/M of the one of the known species and the new center of the low similarity cluster; on the contrary, reassigning one of the known species the low similarity cluster if the difference between the ratio N/M of the one of the known species and the new center of the low similarity cluster is lower than the difference between the ratio N/M of the one of the known species and the center of the high similarity cluster; and
(S77) determining the known species in the high similarity cluster to be the to-be-identified species, and determining the known species in the low similarity cluster not to be the to-be-identified species when the known species reassigned to the high similarity cluster and the known species reassigned to the low similarity cluster are identical to the previous known species assigned to the high similarity cluster and the previous known species assigned to the low similarity cluster of the step (S73);
   wherein when the known species reassigned to the high similarity cluster and the known species reassigned to the low similarity cluster are not identical to the previous known species assigned to the high similarity cluster and the previous known species assigned to the low similarity cluster of the step (S73), the steps of (S74), (S75), and (S76) are repeated until the known species reassigned to the high similarity cluster and the known species reassigned to the low similarity cluster are identical to the previous known species assigned to the high similarity cluster and the previous known species assigned to the low similarity cluster of the step (S76); and then the known species in the high similarity cluster is determined to be the to-be-identified species, and the known species in the low similarity cluster is determined not to be the to-be-identified species.

Please refer to Table 8A and Table 8B, which shows the similarity ratio of each virus type in the database, and the cluster to which the virus type belongs. In the table, the circle "O" represents the high similarity cluster, and the cross "X" represents the low similarity cluster. When the 5 virus types with the highest similarity ratios of patient 1 are taken as an example, they are HPV006, HPV070, HPV075, HPV130, and HPV004, the similarity ratios thereof are respectively 88.89%, 82.61%, 60.87%, 57.89%, and 57.89%. In the steps (S71), HPV075 (60.87%) is randomly selected as the center of the high similarity cluster, and HPV130 (57.89%) is randomly selected as the center of the low similarity cluster. In the step (S72) and (S73), since HPV006 (88.89%), HPV070 (82.61%), and HPV075 (60.87%) are closer to the center (60.87%) of the high similarity cluster, they are assigned to the high similarity cluster; since HPV130 (57.89%) and HPV004 (57.89%) are closer to the center (57.89%) of the low similarity cluster, they are assigned to the low similarity cluster. In the step (S74), the similarity ratio average of the virus types in the high similarity cluster is calculated to be 77.46%, and is used as the new center of the high similarity cluster; the similarity ratio average of the virus types in the low similarity cluster is calculated to be 57.89%, and is used as the new center of the low similarity cluster. In the steps (S75) and (S76), since HPV006 (88.89%) and HPV070 (82.61%) are closer to the new center (77.89%) of the high similarity cluster, they are assigned to the high similarity cluster; since HPV075 (60.87%), HPV130 (57.89%), and HPV004 (57.89%) are closer to the center (57.89%) of the low similarity cluster, they are assigned to the low similarity cluster. In the step (S77), since the virus types assigned to the high similarity cluster and the virus types assigned to the low similarity cluster are not identical to the previous virus types assigned to the high similarity cluster and the previous virus types assigned to the low similarity cluster, the steps of (S74), (S75), and (S76) are repeated. In the step (S74), the similarity ratio average of the virus types in the high similarity cluster is calculated to be 85.75%, and is used as the new center of the high similarity cluster; the similarity ratio average of the virus types in the low similarity cluster is calculated to be 58.88%, and is used as the new center of the low similarity cluster. In the steps (S75) and (S76), since HPV006 (88.89%) and HPV070 (82.61%) are closer to the new center (85.75%) of the high similarity cluster, they are assigned to the high similarity cluster; since HPV075 (60.87%), HPV130 (57.89%), and HPV004 (57.89%) are closer to the center (58.88%) of the low similarity cluster, they are assigned to the low similarity cluster. In step (S77), since the virus types assigned to the high similarity cluster and the virus types assigned to the low similarity cluster are identical to the previous virus types assigned to the high similarity cluster and the previous virus types assigned to the low similarity cluster, the virus types, HPV006 (88.89%) and HPV070 (82.61%), in the high similarity cluster are the virus types possibly carried by patient 1, and the virus types, HPV075 (60.87%), HPV130 (57.89%), and HPV004 (57.89%), in the low similarity cluster are the virus types not carried by patient 1. Thereby, patient 1 is a case with the multiple type infection. The similar method is used to separate the virus types of patient 2 into the two clusters, and there is only one virus type, HPV061 (85%), in the high similarity cluster. Hence, it can be seen that patient 2 is a case with the single type infection.

### Steps of omitting repetition:

Alternatively, the method for the species identification of the present invention may further include steps of omitting repetition, which reduces the interference with the calculation results of the ratio N/M representing the similarity by omitting the comparing steps of the nucleic acid cleavage fragments having low variability in the HPV virus types in the database. The ratios N/M of the virus types having truly high similarities are not significantly affected by the steps of omitting repetition, and on the contrary, the ratios N/M of the virus types having less similarities are substantially reduced. Through the steps, the sensitivity of the similarity ratios between the different virus types is made more significant, and the accuracy of the similarity ratios is improved.

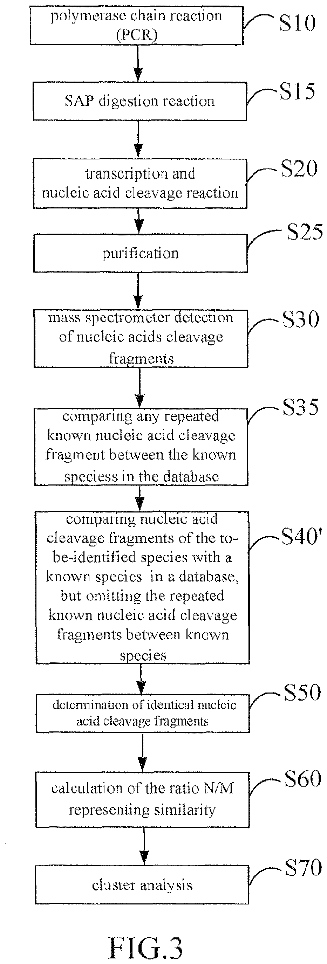

Please refer to FIG. 3, which is a flowchart of a method for species identification including steps of omitting repetition in accordance with another embodiment of the present invention. The embodiment of the steps of omitting repetition is as follows:
(S35) The molecular weight of each of the known nucleic acid cleavage fragments of one of the known virus types prestored in the database is compared with the molecular weight of each of the known nucleic acid cleavage fragments of another similar one of the known virus types prestored in the database prior to the data analysis, so as to determine any repeated known nucleic acid cleavage fragment between the two known species.
(S40') Comparing each of the to-be-tested nucleic acid cleavage fragments of the to-be-identified species with the repeated known nucleic acid cleavage fragment in the data analysis is omitted.

For example, please refer to Table 8A and Table 8B, which show the similarity ratio between each known virus type and the to-be-identified virus type, the cluster to which the virus type belongs, and the similarity ratio after the steps of omitting repetition. In patient 1, the nucleic acid cleavage fragments of the virus type with the highest similarity ratio, HPV006 (88.89%), and the virus type with the second highest similarity ratio, HPV070 (82.61%), are compared to determine the identical/repeated nucleic acid cleavage fragments between the two virus types. When calculating the ratio N/M of HPV006, the nucleic acid cleavage fragments considered as the identical/repeated nucleic acid cleavage fragments are omitted, and the similarity ratio N/M after the steps of omitting repetition is recalculated to be 83.33%. The nucleic acid cleavage fragments of the virus type with the second highest similarity ratio, HPV070 (82.61%), and the virus type with the highest similarity ratio, HPV006 (88.89%), are compared to determine the identical/repeated nucleic acid cleavage fragments between the two virus types. When calculating the ratio N/M of HPV070, the nucleic acid cleavage fragments considered as the identical/repeated nucleic acid cleavage fragments are omitted, and the similarity ratio N/M after the steps of omitting repetition is recalculated to be 76.47%. The nucleic acid cleavage fragments of the virus type with the third highest similarity ratio, HPV075 (60.87%), and the virus type with the highest similarity ratio, HPV006 (88.89%), are compared to determine the identical/repeated nucleic acid cleavage fragments between the two virus types. When calculating the ratio N/M of HPV075, the nucleic acid cleavage fragments considered as the identical/repeated nucleic acid cleavage fragments are omitted, and the similarity ratio N/M after the steps of omitting repetition is recalculated to be 47.06%. The nucleic acid cleavage fragments of the virus type with the fourth highest similarity ratio, HPV130 (57.89%), and the virus type with the highest similarity ratio, HPV006 (88.89%), are compared to determine the identical/repeated nucleic acid cleavage fragments between the two virus types. When calculating the ratio N/M of HPV130, the nucleic acid cleavage fragments considered as the identical/repeated nucleic acid cleavage fragments are omitted, and the similarity ratio N/M after the steps of omitting repetition is recalculated to be 38.46%. The nucleic acid cleavage fragments of the virus type with the fifth highest similarity ratio, HPV004 (57.89%), and the virus type with the highest similarity ratio, HPV006 (88.89%), are compared to determine the identical/repeated nucleic acid cleavage fragments between the two virus types. When calculating the ratio N/M of HPV004, the nucleic acid cleavage fragments considered as the identical/repeated nucleic acid cleavage fragments are omitted, and the similarity ratio N/M after the steps of omitting repetition is recalculated to be 42.86%. The similarity ratio N/M of HPV006 after the steps of omitting repetition is only reduced by 5.56%. The similarity ratio N/M of HPV070 after the steps of omitting repetition is also only reduced by 6.14%, while the similarity ratios N/M of HPV075, HPV130, and HPV004 after the steps of omitting repetition are respectively reduced by 13.81, 19.46, and 15.03%. Therefore, this can be seen that the similarity ratios of the virus types having truly high similarities are affected by the steps of omitting repetition to lesser extents, and are not significantly dropped.

However, in order to simplify the steps of omitting repetition, the steps of omitting repetition can only be performed on the virus types in the high similarity cluster. When there are 2 virus types or more in the high similarity cluster, the steps of omitting repetition are performed on all of the virus types in the high similarity cluster. When there is only 1 virus type or less in the high similarity cluster, it is not required to perform the steps of omitting repetition.

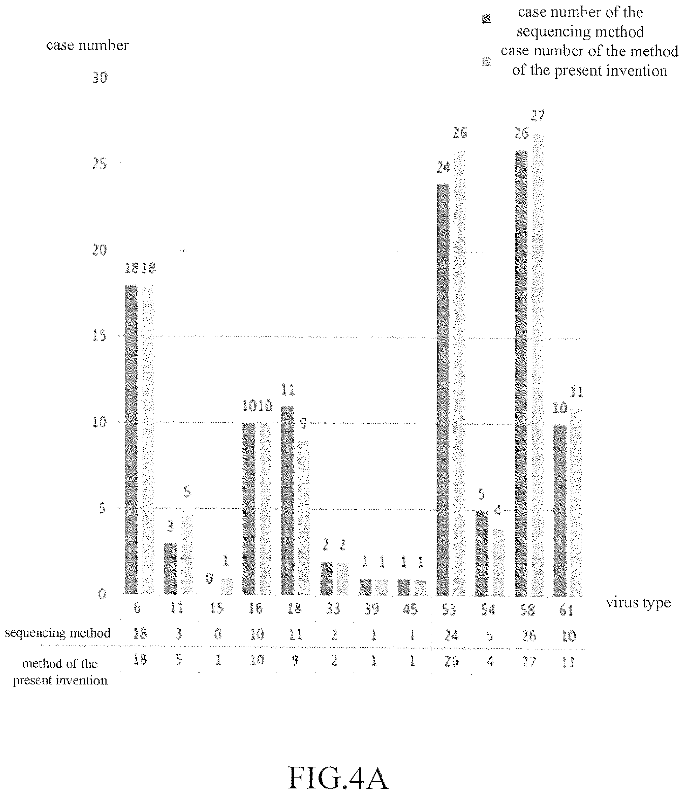

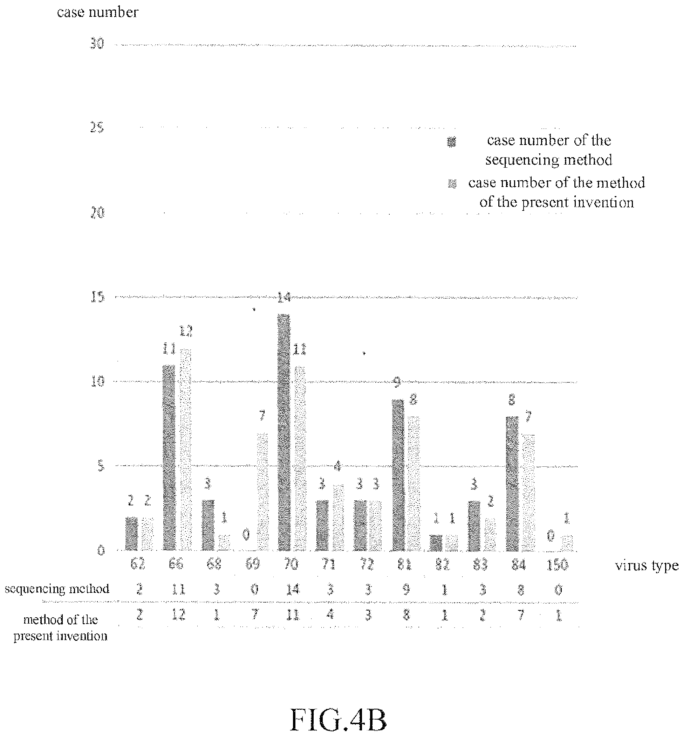

Result comparisons between the method for species identification of the present invention and the conventional sequencing methods:
As shown in FIGs. 4A-4B, which represent the case number of each virus type identified by using a sequencing method and a method for species identification provided by the present invention in the specimens of 168 patients, wherein the numbers on the horizontal axis represent the virus types, the numbers on the vertical axis represent the case numbers, the dark color bars represent the results measured by the sequencing method, and the light color bars represent the results measured by the method for species identification provided by the present invention. Since the method for species identification provided by the present invention can identify the virus types of the multiple type infection, the total case number is 174 and more than 168 of the case number of the sequencing method. The correlation R of the identification results between the method for species identification provided by the present invention and the sequencing method is 0.9675, which shows that the method of the present invention is effective and can replace the sequencing method.

It will be appreciated that the above descriptions are intended only to serve as examples, and that many other embodiments are possible within the scope of the present invention as defined in the appended claims. For instance, the method provided by the present invention is used in different viruses, bacteria or other species of organisms.

## Claims

1. A method for species identification by using molecular weights of nucleic acid cleavage fragments, comprising steps of:
(S10) performing a polymerase chain reaction by using at least a pair of specific primers to amplify a nucleic acid sequence of a to-be-identified species;
(S20) performing a nucleic acid cleavage reaction by using at least a nuclease to cleave the nucleic acid sequence of the to-be-identified species, so as to generate multiple to-be-tested nucleic acid cleavage fragments having different molecular weights
(S30) measuring the molecular weights of the to-be-tested nucleic acid cleavage fragments by using a mass spectrometer;
(S40) comparing the molecular weight of each of the to-be-tested nucleic acid cleavage fragments of the to-be-identified species with molecular weights of multiple known nucleic acid cleavage fragments of a known species prestored in a database;
(S50) determining one of the to-be-tested nucleic acid cleavage fragments to be identical to one of the known nucleic acid cleavage fragments when a difference of the molecular weights between the one of the to-be-tested nucleic acid cleavage fragments and the one of the known nucleic acid cleavage fragments is lower than a specific Dalton value; and
(S60) calculating a ratio N/M of a number N of the to-be-tested nucleic acid cleavage fragments, which are determined to be identical to the known nucleic acid cleavage fragments, relative to a total number M of the known nucleic acid cleavage fragments of the known species, wherein the ratio N/M represents similarity of the nucleic acid sequences between the to-be-identified species and the known species;
wherein the method further comprising the following steps after step (S60):
(S71) randomly selecting a greater ratio N/M from a plurality of the ratios N/M of the multiple known species in the database as a center of a high similarity cluster, and randomly selecting a lower ratio N/M as a center of a low similarity cluster;
(S72) calculating differences between each of the ratios N/M of all of the known species and the center of the high similarity cluster, and differences between each of the ratios N/M of all of the known species and the center of the low similarity cluster;
(S73) assigning one of the known species to the high similarity cluster if the difference between the ratio N/M of the one of the known species and the center of the high similarity cluster is lower than the difference between the ratio N/M of the one of the known species and the center of the low similarity cluster; on the contrary, assigning one of the known species to the low similarity cluster if the difference between the ratio N/M of the one of the known species and the center of the low similarity cluster is lower than the difference between the ratio N/M of the one of the known species and the center of the high similarity cluster;
(S74) calculating an average of the ratios N/M of all of the known species in the high similarity cluster, followed by using the average as the new center of the high similarity cluster; and calculating an average of the ratios N/M of all of the known species in the low similarity cluster, followed by using the average as the new center of the low similarity cluster;
(S75) recalculating the differences between each of the ratios N/M of all of the known species and the new center of the high similarity cluster, and the differences between each of the ratios N/M of all of the known species and the new center of the low similarity cluster;
(S76) reassigning one of the known species to the high similarity cluster if the difference between the ratio N/M of the one of the known species and the new center of the high similarity cluster is lower than the difference between the ratio N/M of the one of the known species and the new center of the low similarity cluster; on the contrary, reassigning one of the known species the low similarity cluster if the difference between the ratio N/M of the one of the known species and the new center of the low similarity cluster is lower than the difference between the ratio N/M of the one of the known species and the new center of the high similarity cluster; and
(S77) determining the known species in the high similarity cluster to be the to-be-identified species, and determining the known species in the low similarity cluster not to be the to-be-identified species when the known species reassigned to the high similarity cluster and the known species reassigned to the low similarity cluster are identical to the previous known species assigned to the high similarity cluster and the previous known species assigned to the low similarity cluster of the step (S73);
wherein when the known species reassigned to the high similarity cluster and the known species reassigned to the low similarity cluster are not identical to the previous known species assigned to the high similarity cluster and the previous known species assigned to the low similarity cluster of the step (S73), the steps of (S74), (S75), and (S76) are repeated until the known species reassigned to the high similarity cluster and the known species reassigned to the low similarity cluster are identical to the previous known species assigned to the high similarity cluster and the previous known species assigned to the low similarity cluster of the step (S76); and then the known species in the high similarity cluster is determined to be the to-be-identified species, and the known species in the low similarity cluster is determined not to be the to-be-identified species;
wherein the number of the known species in the database in step (S40) is more than 2.

2. The method as claimed in claim 1, wherein the specific Dalton value is 2 Daltons.

3. The method as claimed in claim 1, further comprising steps of:
comparing the molecular weight of each of the known nucleic acid cleavage fragments of one of the known species prestored in the database with the molecular weight of each of the known nucleic acid cleavage fragments of another similar one of the known species prestored in the database prior to the step (S40), so as to determine any repeated known nucleic acid cleavage fragment between the two known species; and
omitting comparing each of the to-be-tested nucleic acid cleavage fragments of the to-be-identified species with the repeated known nucleic acid cleavage fragment in the step (S40).

4. The method as claimed in claim 3, wherein the one of the known species and the similar one of the known species are both selected from the high similarity cluster when comparing the molecular weight of each of the known nucleic acid cleavage fragments of the one of the known species prestored in the database with the molecular weight of each of the known nucleic acid cleavage fragments of the similar one of the known species prestored in the database.

5. The method as claimed in claim 1, wherein the nucleic acid sequence is a DNA sequence.

6. The method as claimed in claim 5, further comprising a step of: performing a transcription reaction to transcribe the DNA sequence into an RNA sequence prior to the step (S20).

7. The method as claimed in claim 6, wherein the nuclease is an RNase.

8. The method as claimed in claim 7, wherein the RNase is RNase A, which cleaves the RNA sequence at U sites.

9. The method as claimed in claim 1, wherein the to-be-identified species is a microorganism.

10. The method as claimed in claim 1, wherein the microorganism is a bacterium or a virus.

11. The method as claimed in claim 1, wherein the to-be-identified species is an animal.

12. The method as claimed in claim 1, wherein the to-be-identified species is *Homo sapiens.*

## Patentansprüche

1. Ein Verfahren zur Identifikation von Spezies anhand des Molekulargewichts von Nucleinsäurespaltungsfragmenten, das die folgenden Schritte beinhaltet:
(S10) Durchführen einer Polymerasekettenreaktion anhand von mindestens einem Paar von spezifischen Primern zum Amplifizieren einer Nucleinsäuresequenz einer zu identifizierenden Spezies;
(S20) Durchführen einer Nucleinsäurespaltungsreaktion anhand von mindestens einer Nuclease zum Spalten der Nucleinsäuresequenz der zu identifizierenden Spezies, um dadurch mehrere zu testende Nucleinsäurespaltungsfragmente mit verschiedenen Molekulargewichten zu erzeugen;
(S30) Messen der Molekulargewichte der zu testenden Nucleinsäurespaltungsfragmente mittels Verwendung eines Massenspektrometers;
(S40) Vergleichen des Molekulargewichts jedes der zu testenden Nucleinsäurespaltungsfragmente der zu identifizierenden Spezies mit den Molekulargewichten mehrerer bekannter Nucleinsäurespaltungsfragmente einer bekannten Spezies, die zuvor in einer Datenbank gespeichert wurden;
(S50) Bestimmen eines der zu testenden Nucleinsäurespaltungsfragmente als identisch mit einem der bekannten Nucleinsäurespaltungsfragmente, wenn eine Differenz der Molekulargewichte zwischen dem einen der zu testenden Nucleinsäurespaltungsfragmente und dem einen der bekannten Nucleinsäurespaltungsfragmente geringer als ein spezifischer Dalton-Wert ist; und
(S60) Berechnen eines Verhältnisses N/M einer Zahl N der zu testenden Nucleinsäurespaltungsfragmente, die als identisch mit den bekannten Nucleinsäurespaltungsfragmenten bestimmt werden, relativ zu einer Gesamtzahl M der bekannten Nucleinsäurespaltungsfragmente der bekannten Spezies, wobei das Verhältnis N/M die Ähnlichkeit der Nucleinsäuresequenzen zwischen der zu identifizierenden Spezies und der bekannten Spezies darstellt;
wobei das Verfahren ferner die folgenden Schritte nach Schritt (S60) beinhaltet:
(S71) zufälliges Auswählen eines höheren Verhältnisses N/M aus einer Vielzahl der Verhältnisse N/M der mehreren bekannten Spezies in der Datenbank als ein Mittelpunkt eines Clusters mit großer Ähnlichkeit, und zufälliges Auswählen eines niedrigeren Verhältnisses N/M als ein Mittelpunkt eines Clusters mit geringer Ähnlichkeit;
(S72) Berechnen von Differenzen zwischen jedem der Verhältnisse N/M aller bekannten Spezies und dem Mittelpunkt des Clusters mit großer Ähnlichkeit und von Differenzen zwischen jedem der Verhältnisse N/M aller bekannten Spezies und dem Mittelpunkt des Clusters mit geringer Ähnlichkeit;
(S73) Zuweisen einer der bekannte Spezies zu dem Cluster mit großer Ähnlichkeit, wenn die Differenz zwischen dem Verhältnis N/M einer der bekannten Spezies und dem Mittelpunkt des Clusters mit großer Ähnlichkeit geringer ist als die Differenz zwischen dem Verhältnis N/M einer der bekannten Spezies und dem Mittelpunkt des Clusters mit geringer Ähnlichkeit; im Gegensatz dazu, Zuweisen einer der bekannten Spezies zu dem Cluster mit geringer Ähnlichkeit, wenn die Differenz zwischen dem Verhältnis N/M einer der bekannten Spezies und dem Mittelpunkt des Clusters mit geringer Ähnlichkeit geringer ist als die Differenz zwischen dem Verhältnis N/M einer der bekannten Spezies und dem Mittelpunkt des Clusters mit großer Ähnlichkeit;
(S74) Berechnen eines Durchschnittswerts der Verhältnisse N/M aller bekannter Spezies in dem Cluster mit großer Ähnlichkeit, gefolgt von der Verwendung des Durchschnittswerts als dem neuen Mittelpunkt des Clusters mit großer Ähnlichkeit; und Berechnen eines Durchschnittswerts der Verhältnisse N/M aller bekannten Spezies in dem Cluster mit geringer Ähnlichkeit, gefolgt von der Verwendung des Durchschnittswerts als dem neuen Mittelpunkt des Clusters mit geringer Ähnlichkeit;
(S75) Neuberechnen der Differenzen zwischen jedem der Verhältnisse N/M aller bekannten Spezies und dem neuen Mittelpunkt des Clusters mit großer Ähnlichkeit, und der Differenzen zwischen jedem der Verhältnisse N/M aller bekannten Spezies und dem neuen Mittelpunkt des Clusters mit geringer Ähnlichkeit;
(S76) Neuzuweisen einer der bekannten Spezies zu dem Cluster mit großer Ähnlichkeit, wenn die Differenz zwischen dem Verhältnis N/M einer der bekannten Spezies und dem neuen Mittelpunkt des Clusters mit großer Ähnlichkeit geringer ist als die Differenz zwischen dem Verhältnis N/M einer der bekannten Spezies und dem neuen Mittelpunkt des Clusters mit geringer Ähnlichkeit; im Gegensatz dazu, Neuzuweisen einer der bekannten Spezies zu dem Cluster mit geringer Ähnlichkeit, wenn die Differenz zwischen dem Verhältnis N/M einer der bekannten Spezies und dem neuen Mittelpunkt des Clusters mit geringer Ähnlichkeit geringer ist als die Differenz zwischen dem Verhältnis N/M einer der bekannten Spezies und dem neuen Mittelpunkt des Clusters mit großer Ähnlichkeit; und
(S77) Bestimmen der bekannten Spezies in dem Cluster mit großer Ähnlichkeit als die zu identifizierende Spezies und Bestimmen der bekannten Spezies in dem Cluster mit geringer Ähnlichkeit als die nicht zu identifizierende Spezies, wenn die bekannte Spezies, die dem Cluster mit großer Ähnlichkeit neu zugewiesen wurde, und die bekannte Spezies, die dem Cluster mit geringer Ähnlichkeit neu zugewiesen wurde, identisch mit der vorherigen bekannten Spezies ist, die dem Cluster mit großer Ähnlichkeit zugewiesen wurde, und der vorherigen bekannten Spezies, die dem Cluster mit geringer Ähnlichkeit von Schritt (S73) zugewiesen wurde;
wobei, wenn die bekannte Spezies, die dem Cluster mit großer Ähnlichkeit neu zugewiesen wurde, und die bekannte Spezies, die dem Cluster mit geringer Ähnlichkeit neu zugewiesen wurde, nicht mit der vorherigen bekannten Spezies, die dem Cluster mit großer Ähnlichkeit neu zugewiesen wurde, und mit der vorherigen bekannten Spezies, die dem Cluster mit geringer Ähnlichkeit neu zugewiesen wurde, des Schritts (S73) identisch sind, die Schritte von (S74), (S75) und (S76) wiederholt werden, bis die bekannte Spezies, die dem Cluster mit großer Ähnlichkeit neu zugewiesen wurde, und die bekannte Spezies, die dem Cluster mit geringer Ähnlichkeit neu zugewiesen wurde, mit der vorherigen bekannten Spezies, die dem Cluster mit großer Ähnlichkeit zugewiesen wurde, und mit der vorherigen bekannten Spezies, die dem Cluster mit geringer Ähnlichkeit zugewiesen wurde, des Schritts (S76) identisch sind; und danach wird die bekannte Spezies in dem Cluster mit großer Ähnlichkeit als die zu identifizierende Spezies bestimmt und die bekannte Spezies in dem Cluster mit geringer Ähnlichkeit wird als die nicht zu identifizierende Spezies bestimmt;
wobei die Zahl der bekannten Spezies in der Datenbank in Schritt (S40) mehr als 2 beträgt.

2. Verfahren nach Anspruch 1, wobei der spezifische Dalton-Wert 2 Dalton beträgt.

3. Verfahren nach Anspruch 1, das ferner die folgenden Schritte beinhaltet:
Vergleichen des Molekulargewichts jedes der bekannten Nucleinsäurespaltungsfragmente einer der bekannten Spezies, die zuvor in der Datenbank gespeichert wurden, mit dem Molekulargewicht von jedem der bekannten Nucleinsäurespaltungsfragmente einer anderen ähnlichen der bekannten Spezies, die zuvor in der Datenbank gespeichert wurden, vor dem Schritt (S40), um dadurch ein wiederholtes bekanntes Nucleinsäurespaltungsfragment zwischen den zwei bekannten Spezies zu bestimmen; und
Auslassen des Vergleichens jedes der zu testenden Nucleinsäurespaltungsiragmente der zu identifizierenden Spezies mit dem wiederholten bekannten Nucleinsäurespaltungsfragment in dem Schritt (S40).

4. Verfahren nach Anspruch 3, wobei die eine der bekannten Spezies und die ähnliche der bekannten Spezies beide aus dem Cluster mit großer Ähnlichkeit ausgewählt werden, wenn das Molekulargewicht jedes der bekannten Nucleinsäurespaltungsfragmente einer der bekannten Spezies, die zuvor in der Datenbank gespeichert wurden, mit dem Molekulargewicht jedes der bekannten Nucleinsäurespaltungsfragmente der ähnlichen der bekannten Spezies, die zuvor in der Datenbank gespeichert wurden, verglichen wird.

5. Verfahren nach Anspruch 1, wobei die Nucleinsäuresequenz eine DNA-Sequenz ist.

6. Verfahren nach Anspruch 5, das ferner den folgenden Schritt beinhaltet: Durchführen einer Transkriptionsreaktion zum Transkribieren der DNA-Sequenz in eine RNA-Sequenz vor dem Schritt (S20).

7. Verfahren nach Anspruch 6, wobei die Nuclease eine RNase ist.

8. Verfahren nach Anspruch 7, wobei die RNase RNase A ist, die die RNA-Sequenz an U-Stellen spaltet.

9. Verfahren nach Anspruch 1, wobei die zu identifizierende Spezies ein Mikroorganismus ist.

10. Verfahren nach Anspruch 1, wobei der Mikroorganismus eine Bakterie oder ein Virus ist.

11. Verfahren nach Anspruch 1, wobei die zu identifizierende Spezies ein Tier ist.

12. Verfahren nach Anspruch 1, wobei die zu identifizierende Spezies Homo sapiens ist.

## Revendications

1. Procédé d'identification d'une espèce au moyen des masses moléculaires de fragments de clivage d'un acide nucléique, comprenant des étapes consistant à :
(S10) effectuer une réaction en chaîne par polymérase en utilisant au moins une paire d'amorces spécifiques pour amplifier une séquence d'un acide nucléique d'une espèce à identifier ;
(S20) effectuer une réaction de clivage de l'acide nucléique en utilisant au moins une nucléase pour cliver la séquence de l'acide nucléique de l'espèce à identifier afin de produire de multiples fragments de clivage de masses moléculaires différentes de l'acide nucléique à tester ;
(S30) mesurer les masses moléculaires des fragments de clivage de l'acide nucléique à tester en utilisant un spectromètre de masse ;
(S40) comparer la masse moléculaire de chacun des fragments de clivage de l'acide nucléique à tester de l'espèce à identifier aux masses moléculaires de multiples fragments de clivage d'un acide nucléique connu d'une espèce connue déjà répertoriée dans une banque de données ;
(S50) déterminer que l'un des fragments de clivage de l'acide nucléique à tester est identique à l'un des fragments de clivage de l'acide nucléique connu quand une différence entre les masses moléculaires du fragment de clivage de l'acide nucléique à tester et du fragment de clivage de l'acide nucléique connu est inférieure à une valeur spécifique en Daltons ; et
(S60) calculer un rapport N/M, où N est le nombre de fragments de clivage de l'acide nucléique à tester qui ont été caractérisés comme étant identiques à des fragments de clivage de l'acide nucléique connu et M est le nombre total de fragments de clivage de l'acide nucléique connu d'une espèce connue, un rapport N/M représentant la similarité entre des séquences d'un acide nucléique de l'espèce à identifier et de l'espèce connue
le procédé comprenant en outre, après l'étape (S60), les étapes consistant à :
(S71) sélectionner de manière aléatoire un rapport N/M plus élevé parmi une pluralité de rapports N/M de multiples espèces connues répertoriées dans la banque de données en tant que centre d'un groupe de forte similarité et sélectionner de manière aléatoire un rapport N/M plus faible en tant que centre d'un groupe de faible similarité ;
(S72) calculer les différences entre chacun des rapports N/M de toutes les espèces connues et le centre du groupe de forte similarité et les différences entre chacun des rapports N/M de toutes les espèces connues et le centre du groupe de faible similarité ;
(S73) assigner une des espèces connues au groupe de forte similarité si la différence entre le rapport N/M de la une des espèces connues et le centre du groupe de forte similarité est inférieure à la différence entre le rapport N/M de la une des espèces connues et le centre du groupe de faible similarité ; et inversement assigner une des espèces connues au groupe de faible similarité si la différence entre le rapport N/M de la une des espèces connues et le centre du groupe de faible similarité est inférieure à la différence entre le rapport N/M de la une des espèces connues et le centre du groupe de forte similarité ;
(S74) calculer une moyenne des rapports N/M de toutes les espèces connues assignées au groupe de forte similarité puis utiliser la moyenne en tant que nouveau centre du groupe de forte similarité ; et calculer une moyenne des rapports N/M de toutes les espèces connues assignées au groupe de faible similarité puis utiliser la moyenne en tant que nouveau centre du groupe de faible similarité ;
(S75) recalculer les différences entre chacun des rapports N/M de toutes les espèces connues et le nouveau centre du groupe de forte similarité et les différences entre chacun des rapports N/M de toutes les espèces connues et le nouveau centre du groupe de faible similarité ;
(S76) réassigner une des espèces connues au groupe de forte similarité si la différence entre le rapport N/M de la une des espèces connues et le nouveau centre du groupe de forte similarité est inférieure à la différence entre le rapport N/M de la une des espèces connues et le nouveau centre du groupe de faible similarité ; et inversement réassigner une des espèces connues au groupe de faible similarité si la différence entre le rapport N/M de la une des espèces connues et le nouveau centre du groupe de faible similarité est inférieure à la différence entre le rapport N/M de la une des espèces connues et le nouveau centre du groupe de forte similarité ;
(S77) déterminer que l'espèce connue dans le groupe de forte similarité est l'espèce à identifier et déterminer que l'espèce connue dans le groupe de faible similarité n'est pas l'espèce à identifier quand l'espèce connue réassignée au groupe de forte similarité et l'espèce connue réassignée au groupe de faible similarité sont identiques à l'espèce connue précédente assignée au groupe de forte similarité et à l'espèce connue précédente assignée au groupe de faible similarité à l'étape (S73) ;
où quand l'espèce connue réassignée au groupe de forte similarité et l'espèce connue réassignée au groupe de faible similarité ne sont pas identiques à l'espèce connue précédente assignée au groupe de forte similarité et à l'espèce connue précédente assignée au groupe de faible similarité à l'étape (S73), les étapes (S74), (S75) et (S76) sont répétées jusqu'à ce que l'espèce connue réassignée au groupe de forte similarité et l'espèce connue réassignée au groupe de faible similarité soient identiques à l'espèce connue précédente assignée au groupe de forte similarité et à l'espèce connue précédente assignée au groupe de faible similarité à l'étape (S76) ; l'espèce connue dans le groupe de forte similarité étant alors déterminée comme étant l'espèce à identifier et l'espèce connue dans le groupe de faible similarité étant déterminée comme n'étant pas l'espèce à identifier ;
où le nombre d'espèces connues dans la banque de données à l'étape (S40) est supérieur à 2.

2. Procédé tel que revendiqué à la revendication 1, où la valeur spécifique en Daltons est 2 Daltons.

3. Procédé tel que revendiqué à la revendication 1, comprenant en outre les étapes consistant à :
comparer la masse moléculaire de chacun des fragments de clivage d'un acide nucléique connu d'une espèce connue déjà répertoriée dans la banque de données à la masse moléculaire de chacun des fragments de clivage d'un acide nucléique connu d'une autre espèce connue similaire déjà répertoriée dans la banque de données avant l'étape (S40) afin d'identifier toute répétition d'un fragment de clivage de l'acide nucléique entre les deux espèces connues ; et
omettre la comparaison de chacun des fragments de clivage de l'acide nucléique à tester de l'espèce à identifier avec le fragment de clivage répété de l'acide nucléique connu à l'étape (S40).

4. Procédé tel que revendiqué à la revendication 3, où la une des espèces connues et celle des espèces connues qui est similaire sont l'une et l'autre sélectionnées dans le groupe de forte similarité à la comparaison entre les masses moléculaires de chacun des fragments de clivage de l'acide nucléique connu de l'une des espèces connues déjà répertoriée dans la banque de données et la masse moléculaire de chacun des fragments de clivage de l'acide nucléique connu de celle des espèces connues qui est similaire déjà répertoriée dans la banque de données.

5. Procédé tel que revendiqué à la revendication 1, où la séquence d'acide nucléique est une séquence d'ADN.

6. Procédé tel que revendiqué à la revendication 5, comprenant en outre l'étape consistant à : effectuer une réaction de transcription pour transcrire la séquence d'ADN en une séquence d'ARN avant l'étape (S20).

7. Procédé tel que revendiqué à la revendication 6, où la nucléase est une RNase.

8. Procédé tel que revendiqué à la revendication 7 où la RNase est une RNase A, qui clive la séquence d'ARN au niveau de résidus U.

9. Procédé tel que revendiqué à la revendication 1, où l'espèce à identifier est un micro-organisme.

10. Procédé tel que revendiqué à la revendication 1, où le micro-organisme est une bactérie ou un virus.

11. Procédé tel que revendiqué à la revendication 1, où l'espèce à identifier est un animal.

12. Procédé tel que revendiqué à la revendication 1, où l'espèce à identifier est *Homo sapiens.*
